(19) Europäisches Patentamt
European Patent Office
Office européen des brevets

(11) Publication number: **0 300 664 A2**

# (12) EUROPEAN PATENT APPLICATION

(21) Application number: **88306353.9**

(51) Int. Cl.4: **C07D 501/46 , A61K 31/545**

(22) Date of filing: **12.07.88**

Claims for the following Contracting States: ES + GR.

(30) Priority: **23.07.87 EP 87401719**

(43) Date of publication of application:
**25.01.89 Bulletin 89/04**

(84) Designated Contracting States:
**AT BE CH DE ES FR GB GR IT LI LU NL SE**

(71) Applicant: **I.C.I.- PHARMA**
**Immeuble "Le Galien", 1, Rue des Chauffours B.P. 127**
**F-95022 Cergy Cedex(FR)**

(72) Inventor: **Jung, Frederick Henri**
**Rue de Moulin Cliquot**
**Taissy F-51500 Rilly la Montagne(FR)**

(74) Representative: **Denerley, Paul Millington, Dr.**
**Imperial Chemical Industries PLC Legal Department: Patents P.O. Box 6, Bessemer Road**
**Welwyn Garden City Hertfordshire AL7 1HD(GB)**

## (54) Cephalosporin compounds.

(57) Cephalosporins having the formula:

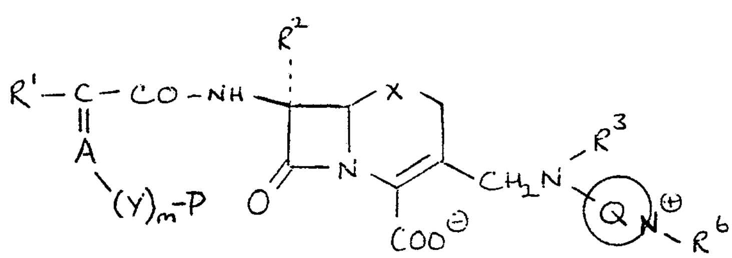

are described, wherein X is S, SO, $CH_2$ or O, $R^2$ is hydrogen, methoxy or formamido, $R^3$ is hydrogen, alkenyl, alkyl or substituted alkyl, Q is a mono- or bicyclic heterocyclic ring, variously substituted, $R^1$ represents various groups including 2-aminothiazol-4-yl, A is =NO-, =N-NH- or =CH-, Y is CO or various linking groups, m is zero or one and P is a benzene ring with two ortho groups, one of which is hydroxy or an in vivo hydrolysable ester thereof and the other is hydroxy, an in vivo hydrolysable ester thereof, carboxy, sulpho, hydroxymethyl, $-NHSO_2CH_3$ or $-NHCONH_2$; or P is a particularly substituted pyridone or pyranone. The use of such compounds as antibacterial agents is described as are processes for their preparation.

EP 0 300 664 A2

# CEPHALOSPORIN COMPOUNDS

This invention relates to cephalosporin derivatives which have antibacterial activity.

The cephalosporin derivatives referred to herein are named in accordance with the "cephem" nomenclature and numbering system proposed in J. Amer. Chem. Soc 1962. 84. 3400.

Formulae referred to by roman numerals are set out hereinafter.

European Patent Application 164944 describes cephalosporin derivatives having various heterocyclic aminomethyl substituents at the 3-position. The compounds of the present invention represent a class of novel cephalosporins related to those of EP-A 164944 and having particularly good antibacterial activity, especially against Pseudomonas strains.

According to the invention there is provided a cephalosporin derivative having antibacterial activity of the formula I in which X is sulphur, oxygen, methylene or sulphinyl (R or S configuration);

R1 is 2-aminothiazol-4-yl or 2-aminooxazol-4-yl each optionally substituted in the 5-position by fluorine, chlorine or bromine, or R1 is 5-amino-isothiazol-3-yl, 5-amino-1,2,4-thiadiazol-3-yl, 3-aminopyrazol-5-yl, 3-aminopyrazol-4-yl, 2-amino-pyrimidin-5-yl, 2-aminopyrid-6-yl, 4-aminopyrimidin-2-yl, 2-amino-1,3,4-thiadiazol-5-yl or 5-amino-1-methyl-1,2,4-triazol-3-yl;

R2 is hydrogen, methoxy or formamido;

R3 is hydrogen, (1-4C)alkyl, halo(1-4C)alkyl, hydroxy(1-4C)alkyl, (1-4C)alkoxy(1-4C)alkyl, carboxy (1-4C)-alkyl, amino(1-4C)alkyl, cyano(1-4C)alkyl, (1-4C) alkanoylamino(1-4C)alkyl, (3-6C)alkenyl, phenyl(1-4C)alkyl or heteroaryl(1-4C)alkyl wherein heteroaryl is a 5- or 6- membered ring containing 1, 2 or 3 heteroatoms selected from nitrogen, oxygen and sulphur;

A is of the formula =NO-, =N-NH- or =CH-;

Y is -CO- or a group of formula

$$-(CH_2)_n-\overset{R^a}{\underset{R^b}{\overset{|}{\underset{|}{C}}}}-(CH_2)_{n'}-(Y')_p-$$

(wherein Ra and Rb are hydrogen or (1-3C)alkyl or together represent (3-7C)cycloalkyl or (1-8C)alkylidene, n and n′ are independently 0-3, p is 0 or 1 and Y′ is -CO-, or (when A is of the formula =NO-) Y′ may additionally be selected from -CONH-, -NHCO-, -C(=O)O-, -OC(=O)-, $-NHSO_2$, $-SO_2NH-$, -O-, -S- and $-NR^c-$ where $R^c$ is hydrogen, (1-4C)alkyl or (2-4C)alkenyl) the said group being optionally substituted by carboxy;

m is 0 or 1 such that when m=0 A is connected directly to ring P by a covalent bond;

P represents:

(i) a benzene ring, optionally fused to a further benzene ring (so forming a naphthyl group) or to a 5 or 6 membered heterocyclic aromatic group containing 1, 2 or 3 heteroatoms selected from nitrogen, oxygen and sulphur and said ring system substituted by groups W and Z which are <u>ortho</u> with respect to one another wherein W is hydroxy or an in vivo hydrolysable ester thereof and Z is hydroxy, an in vivo hydrolysable ester thereof, carboxy, sulpho, hydroxymethyl, $-NHSO_2CH_3$ or $-NHCONH_2$;

(ii) a group of formula (II) or

(iii) a group of formula (III) and tautomers thereof wherein M represents oxygen or $NR^d$ represents hydrogen or (1-4C)alkyl,

ring system P optionally being further substituted by (1-4C)alkyl, halogen, hydroxy, cyano, trifluoromethyl, nitro, amino, mono- or di-(1-4C)alkylamino, formyl, (1-4C)alkanoyl, (1-4C)alkoxy, (1-4C)alkylthio, (1-4C)-alkanoyloxy, carbamoyl or mono- or di(1-4C)alkyl-carbamoyl;

Q represents a 5 to 10 membered mono- or bicyclic heterocyclic ring system bonded to NR3 via a carbon atom and containing, in addition to the positively charged nitrogen atom, 0 to 5 further heteroatoms selected from nitrogen, oxygen and sulphur and being optionally substituted:

on a carbon atom or atoms available for substitution by 1,2 or 3 groups R4 wherein R4 is halogen, (1-6C)-alkyl, carboxy, (2-6C)alkoxycarbonyl, (2-6C)alkoxycarbonyl(1-4C)alkyl, (1-6C)alkoxy, (1-6C)alkylthio, cyano,

(1-4C) cyanoalkyl, amino, (1-6C)alkylamino, (2-8C)dialkylamino, phenyl(1-4C)alkylamino, nitrophenyl(1-4C)-alkylamino, heteroaryl(1-4C)alkyl (wherein heteroaryl is as hereinbefore defined), (3-6C)alkenylamino, amino(1-6C)alkylamino, (1-6C)alkoxy(1-6C)alkylamino, hydroxy(1-6C)alkylamino, hydroxy, mercapto, carbamoyl, (2-6C) alkylcarbamoyl, (3-10C)dialkylcarbamoyl, phenylthio and heteroarylthio (wherein heteroaryl is hereinbefore defined) wherein when more than one group R4 is present these may be the same or different;

on an uncharged nitrogen atom available for substitution by a group R5 wherein R5 is (1-6C)alkyl, phenyl or benzyl;

R6 is hydrogen, (1-6C)alkyl (optionally substituted by carboxy, (1-6C)alkoxycarbonyl, carbamoyl, mono- or di-(1-4C)alkylcarbamoyl, hydroxy, (1-4C)alkoxy, amino, mono- or di-(1-4C)alkylamino, (1-4C)alkanoyl, benzoyl, cyano, carboxyaminocarbonyl, (1-6C)alkoxy-carbonylaminocarbonyl, (1-4C)alkoxy(2-4C)a'koxy or phenyl), (1-6C)alkoxy, phenyl(1-6C)alkoxy, amino, (1-6C)alkylamino, (3-6C)cycloalkyl, (3-6C)cycloalkenyl, cyano(3-6C)cycloalkenyl, (2-6C)alkenyl (optionally substituted by (1-6C)alkyl, halogen, cyano, carbamoyl, mono- or di-(1-4C)alkylcarbamoyl, piperidinocarbonyl or morpholinocarbonyl), 2-ureidoethyl, 2-thioureidoethyl, 2-(thioacetylamino)ethyl, sulphamoyl, 2-amino-2-carboxyethyl, acetylaminomethyl, phthalimidomethyl, 4-5-dihydroimidazol-2-yl-methyl, 3,4,5,6-tetrahydropyrimidin-2-ylmethyl, 2-(1,2,3,6-tetrahydro- 2,6-dioxopurin-7-ylethyl, 2-hydroxyiminopropyl (syn or anti) [(1-4C)alkoxyimino]propyl (syn or anti) or phenyl,

or R6 is of the formula $-(CH_2)_2-NR7R8R9$ in which R7, R8 and R9 are (1-4C)alkyl,

or R6 is of the formula-$(CH_2)_s$-R10 in which s is 0-2 and R10 is pyridine, pyridazine, pyrimidine, pyrazine 1,2,5,6-dihydro-5,6-dioxo-1,2,4-triazine, 2-[(1-4C)alkyl]-1,2,5,6-dihydro-5,6-dioxo-1,2,4-triazine, 1-[(1-4C)-alkyl]tetrazole, furan, thiophene, pyrrole, 1-[(1-4C)alkyl]pyrrole, oxazole, thiazole, imidazole, 1-[1-4C)alkyl]-imidazole, isoxazole, isothiazole, pyrazole, 1,2,3-thiadiazole, 1-[(1-4C)alkyl]pyrazole, benzfuran, benzthiophene, indole, oxindole, 1-[(1-4C)alkyl]indole, benzoxazole, benzthiazole, benzimidazole, 1-[(1-4C)alkyl]-benzimidazole, or 3,4-dihydro-4-oxo-2H-benzo[e]oxazine (each of these ring systems being linked to $(CH_2)_s$ through carbon and each ring system being optionally substituted by halogen, (1-6C)alkyl, (1-4C)haloalkyl, (3-6C)cycloalkyl, (3-6C)cycloalkyl(1-4C)alkyl, (2-6C)alkenyl, carboxy, (2-6C)alkoxycarbonyl, (1- 6C)alkoxy, cyano, (2-6C)cyanoalkenyl, carbamoyl, mono-or di-(1-4C)-alkylcarbamoyl, (1-4C)alkanoylamino, guanidino, hydroxy, nitro or amino),

or R6 is 2-guanidino-thiazol-4-ylmethyl, hydroxybenzoylmethyl, 2-thenyl, 2-imidazolylmethyl or cinnamyl (each optionally substituted by halogen, (1-6C)alkyl, hydroxy, (1-4C)alkoxy, carboxy, (2-6C)alkoxycarbonyl, nitro or carbamoyl),

or R6 is $-(CH_2)_tNHCOR11$ or $-(CH_2)_tS(O)_u-R11$ in which t is 1-6, u is 0, 1 or 2 and R11 is (1-6C)alkyl or (1-6C)alkoxy,

or R6 is of the formula $(CH_2)_2N=CR12NR13R14$ or

$-(CH_2)_vC(=NR12)NR13R14$ or a tautomer thereof in which v s 1-6 and R12, R13, R14 are hydrogen or (1-4C)alkyl,

(wherein when R6 is or contains a phenyl group, the phenyl group is optionally substituted by 1 or 2 groups selected from halogen, (1-6C)alkyl, (1-6C)alkoxy, amino, carboxy, nitro, carbamoyl, cyano, trifluoromethyl, aminomethyl, (1-4C)alkanoyl, (1-4)alkanoylamino, halo(1-4C)alkyl, (2-6C)alkoxycarbonyl, mono- or di-(1-4C)-alkylcarbamoyl, mesyl, vinyl, sulpho, sulphamoyl or mono- or di(1- 4C)alkylsulphamoyl);

or, where the positively charged nitrogen atom in ring Q occurs at the junction between fused rings, R6 is absent;

and the N-oxides thereof where chemically possible;

salts formed with acids and bases which afford pharmaceutically acceptable anions and cations respectively.

It is to be understood that in the above formula I and throughout this specification, the illustrated stereochemistry of the ceph-3-em nucleus, and its optional modifications at the 1-position, is the absolute configuration. It is also to be understood that, since ring Q contains a quaternary nitrogen, the compounds of the formula I will normally exist in zwitterionic form, involving the quaternary nitrogen and the carboxy group. When the compound of the formula I contains further acidic or basic substituents, it is to be understood that the possibility of a double zwitterionic form of the compound will arise. Alternatively, exogenous anions or cations may be included, to form pharmaceutically-acceptable base-addition or acid-addition salts, as defined above.

It will also be understood that references herein to a particular nitrogen atom bearing a positive charge and to other nitrogen atoms being uncharged are made for the sake of convenience in defining the compounds of the invention and that all possible resonance hybrids of the structures so defined are included within the scope of the invention.

A particular meaning for R2 is hydrogen.

A particular meaning for X is sulphur.

Particular meanings for R3 are hydrogen, methyl, ethyl, n-propyl, isopropyl, 2-fluoroethyl, 2-chloroethyl, 2-hydroxymethyl, 2-methoxyethyl, carboxymethyl, (R) and (S)-1-carboxyethyl, 2-aminoethyl, 2-cyanoethyl, 2-formamidoethyl, allyl, furfuryl, benzyl or 4-pyridylmethyl.

A particular ring system represented by ring Q is a 5 or 6 membered ring containing the positively charged nitrogen atom and 0 to 3 further heteroatoms selected from nitrogen, oxygen and sulphur and optionally fused on an available carbon- carbon or carbon-nitrogen bond to a benzene ring or to a ring of formula (IV) wherein T, U and V are selected from oxygen, sulphur, nitrogen, carbon -NH- and -CH- and optionaly substituted where possible, by one or more groups R4 and R5 as hereinbefore defined.

Further particular ring systems represented by ring Q are those of formulae V-XII wherein one of D, D', E and E' represents NR6, the remainder representing uncharged nitrogen atoms.

Still further particular ring systems represented by ring Q are those of formula XIII and XIV wherein R17 represents hydrogen or a group R5 as hereinbefore defined, and XV-XIX.

Still further particular ring systems represented by ring Q are those of formulae XX (wherein T is as hereinbefore defined) and XXI-XXIV.

Any of rings V-XXIV may optionally be substituted, where possible, by one or more groups R4 and R5 as hereinbefore defined.

When ring Q represents a 5- or 6- membered ring as defined above a particular meaning therefor is pyridine or pyrimidine, optionally fused to a benzene ring or a ring of formula IV which is a thienyl group.

Particular meanings for the group R4 are fluorine, chlorine, bromine, methyl, ethyl, n-propyl, isopropyl, carboxy, methoxycarbonyl, ethoxycarbonyl, methoxycarbonylmethyl, ethoxycarbonylmethyl, methoxy, ethoxy, methylthio, ethylthio, cyano, cyanomethyl, 2-cyanoethyl, amino, methylamino, ethylamino, isopropylamino, dimethylamino, benzylamino, nitrobenzylamino, allylamino, 2-aminoethylamino, 2-methoxyethylamino, 2-hydroxy-ethylamino, hydroxy, mercapto, carbamoyl, methyl-carbamoyl, ethylcarbamoyl, dimethylcarbamoyl, phenylthio and heteroarylthio in which the heteroaryl ring is a furan, thiophene, imidazole, thiazole, pyrazole, thiadiazole, pyridine, pyrimidine, pyrazine or pyridazine.

Particular meanings for the group R5 are methyl, ethyl, n-propyl, isopropyl, phenyl or benzyl.

Particular meanings for R17 are hydrogen and the particular meanings given above for R5.

Particular meanings for the group R6 are hydrogen, methyl, ethyl, n-propyl, isopropyl, t-butyl, carboxymethyl, 2-carboxyethyl, methoxycarbonylmethyl, 2-methoxycarbonylethyl, carbamoylmethyl, 2-carbamoylethyl, mono- and dimethylcarbamoylmethyl, hydroxymethyl, 2-hydroxyethyl, methoxymethyl, 2-methoxyethyl, aminomethyl, 2-aminoethyl, 2-methylaminoethyl, 2-dimethylaminoethyl, acetylmethyl, propionylmethyl, benzoylmethyl, cyanomethyl, 2-cyanoethyl, 3-cyanopropyl, carboxyaminocarbonylmethyl, 2-(carboxyaminocarbonyl)ethyl, methoxycarbonylaminocarbonylmethyl, 2-(methoxycarbonylaminocarbonyl)-ethyl, 2-methoxy-ethoxymethyl, benzyl, 2-phenethyl, methoxy, ethoxy, benzyloxy, 2-phenylethoxy, amino, methylamino, ethylamino, cyclopropyl, cyclobutyl, cyclopentyl, cyclopentenyl, 3-cyanocyclopent-2-enyl, vinyl, allyl, 2,4-pentadienyl, 3-chloroallyl (cis and trans), 3-cyanoallyl, 2-ureidoethyl, 2-thioureidoethyl, 2-thioacetylamino)ethyl, sulphamoyl, 2-amino- 2-carboxyethyl, acetylaminomethyl, phthalimidomethyl, 4,5-dihydroimidazol-2-ylmethyl, 3,4,5,6-tetra-hydropyrimidin-2-ylmethyl, 2-(1,2,3,6-tetrahydro-2,6-dioxopurin-7-yl)ethyl, 2-hydroxyiminopropyl (syn or anti), 2-(methoxyimino)propyl (syn or anti), 2-(ethoxyimino)propyl (syn or anti) or phenyl,

or where R6 is of the formula $-(CH_2)_2-NR7R8R9$ in which R7, R8 and R9 are methyl or ethyl,

or where R6 is of the formula $-(CH_2)s-R10$ in which s is 0-2 and R10 is pyridine, pyridazine, pyrimidine, pyrazine, 1,2,5,6-dihydro-5,6-dioxo-1,2,4-triazine, 2-(methyl or ethyl)-1,2,5,6-dihydro-5,6-dioxo-1,2,4-triazine, 1-(methyl or ethyl)tetrazole, furan, thiophene, pyrrole, 1-(methyl or ethyl)pyrrole, oxazole, thiazole, imidazole, 1-(methyl or ethyl)imidazole, isoxazole, isothiazole, pyrazole, 1-(methyl or ethyl) pyrazole, 1,2,3-thiadiazole, benzfuran, benzthiophene, indole, 1-(methyl or ethyl)indole, oxindole, benzoxazole, benzthiazole, benzimidazole, 3,4-dihydro-4-oxo-2H-benzo[e]oxazine, 1-(methyl or ethyl)benzimidazole, each of these ring systems being linked to $(CH_2)_s$ through carbon and each ring system being optionally substituted by fluorine, chlorine, bromine, methyl, ethyl, n-propyl, isopropyl, trifluoromethyl, cyclopropylmethyl, formamido, carboxy, methoxycarbonyl, ethoxycarbonyl, methoxy, ethoxy, cyano, 3-cyanoallyl, carbamoyl, methylcarbamoyl, dimethylcarbamoyl, hydroxy, guanidino, nitro or amino,

or where R6 is 2-guanidinothiazol-4-ylmethyl, 3-hydroxybenzoylmethyl, 2-thenyl, 2-imidazolylmethyl or cinnamyl, each optionally substituted by fluorine, chlorine, bromine, methyl, ethyl, n-propyl, isopropyl, hydroxy, methoxy, ethoxy, carboxy, methoxycarbonyl, ethoxycarbonyl or carbamoyl,

or where R6 is of the formula $-(CH_2)_t-NH-CO-R11$ or $(CH_2)_t-S(O)_s-R11$ in which t is 1-6, s is 0, 1 or 2 and R11 is methyl, ethyl, methoxy or ethoxy,

or where R6 is of the formula $(CH_2)_2N=CR12NR13R14$ or $(CH_2)_2C(=NR12)NR13R14$ in which R12, R13 and R14 are hydrogen or methyl,
(wherein when R6 is or contains a phenyl group the phenyl group is optionally substituted by 1 or 2 groups selected from fluorine, chlorine, bromine, amino, carboxy, nitro, carbamoyl, cyano, trifluoromethyl, aminomethyl, methyl, ethyl, methoxy, ethoxy, acetyl, acetamido, methoxycarbonyl, ethoxycarbonyl, methylcarbamoyl, dimethylcarbamoyl, diethylcarbamoyl, mesyl and sulpho).

In a particularly preferred aspect $R^1$ is 2-aminothiazol-4-yl. Preferably A is a group =NO- (in the syn form). In a particular aspect Y is a group CO when m is one. In a further particular aspect Y is a group $-(CH_2)_nCR^aR^b(CH_2)_{n'}-(Y')_p-$ wherein n, n′, p, $R^a$, $R^b$ and Y′ are as hereinbefore defined. Preferably $R_a$ and $R_b$ are independently hydrogen or methyl or together represent cyclopropyl, cyclobutyl or cyclopentyl. Preferably n is zero. Preferably n′ is zero or one. In a particular aspect $-(Y')_p-$ is -CO-, -CONH-, -NHCO-, -COO- or -OCO-; preferably $-(Y')_p-$ is -COO- or -CO-. Particular examples of $-(Y)_m-$ include -CO- and $C_{1-4}$ alkylene optionally substituted by carboxy, such as $-CH_2-$, $-CH_2CH_2-$, $-CH(CH_3)CH_2-$and -CH(COOH)-.

Preferably P is a benzene ring substituted by groups W and Z and optionally further substituted by halo for example bromo. W is hydroxy or an in vivo hydrolysable ester thereof. In vivo hydrolysable esters are those pharmaceutically acceptable esters that hydrolyse in the human or animal body to produce the parent hydroxy compound. Such esters can be identified by administering e.g. intravenously to a test animal the compound under test and subsequently examining the test animal's body fluids. Suitable in vivo hydrolysable esters include $C_{1-6}$ alkanoyloxy for example acetoxy, propionyloxy and pivaloyloxy, $C_{1-4}$ alkoxycarbonyloxy for example ethoxycarbonyloxy and phthalidyloxy. Preferable Z is hydroxy or an in vivo hydrolysable ester thereof. Conveniently W and Z have the same value and are both hydroxy or both in vivo hydrolysable esters for example they are both acetoxy or pivaloyloxy.

A particular acid which affords a pharmaceutically-acceptable anion is, for example, hydrochloric, hydrobromic, phosphoric, sulphuric, citric or maleic acid.

A particular base which affords a pharmaceutically acceptable cation is, for example, a base containing an alkali metal, (e.g.sodium or potassium) or an alkaline earth metal (e.g. calcium or magnesium), or a primary, secondary or tertiary organic amine (e.g. triethylamine, morpholine, N-methylpiperidine. N-ethylpiperidine, procaine, dibenzylamine or N,N′-dibenzylethylenediamine), or other amine which has been used to form salts with cephalosporins.

The compounds of formula I may be prepared by the following processes, which form further aspects of the invention, and in which the groups R1, A, R2, R3, R6, Q, P, Y, X and m have the meanings already assigned to them.

(A) Reaction of a compound of formula XXV (wherein L is a leaving group) with a nucleophilic compound serving to form the desired group $-NR3-Q-R^6$.

A nucleophilic compound serving to form the group -NR3-Q may be for example a compound of formula XXVI or XXVII. Particular meanings for the leaving group L include $C_{1-4}$-alkanoyloxy (e.g. acetoxy) and halo (e.g. chloro or iodo).

(B) Reaction of a compound of formula XXVIII with a compound of formula XXIX (wherein R18 is a leaving group).

Particular meanings for the leaving group R18 include (1-4C)alkylthio (eg methylthio), (1-4C)-alkylsulphoxy (eg methylsulphoxy) and halogen (eg chlorine).

(C) Reaction of a compound of formula XXX with a compound of formula XXXI wherein J and K in the above formulae XXX and XXXI are such that reaction takes place to form the link $-(Y)_m-$between A and the ring P.

Particular meanings for J and K include for example where A is =N-O-, J=H and K=COCl, which react to form a link $-(Y)_m$ of formula -0.CO-.

(D) (Where a compound in which W and Z are hydroxy groups is desired) deprotection of a corresponding compound in which hydroxy groups W and Z are protected by hydroxy protecting groups.

(E) (Where a compound in which W and Z are in vivo hydrolysable ester groups is required) reaction of a compound in which W and Z are hydroxy groups with an appropriate acid or a reactive derivative thereof. Particular in vivo hydrolysable esters are (1-4C)alkanoyloxy groups. A reactive derivative of a (1-4C)alkanoic acid may be for example the acid chloride.

(F) (Where a compound of formula I having a free carboxy group is required) deprotection of a corresponding compound containing a protected carboxy group.

(G) (Where a salt of the compound of formula I is required) reaction of a compound of formula I having a free acidic or basic group with a pharmaceutically acceptable base or acid.

(H) (Where a compound of formula I having a free amino group is required) deprotection of a corresponding compound having a protected amino group.

(I) (Where a compound of formula i which is a cephalosporin 1-oxide is required) oxidation of the corresponding 1-S compound by conventional means.

(J) (Where a compound of formula I having S at the 1-position is required) reduction of the corresponding 1-oxide by conventional means.

(K) Reaction of a compound of formula XXXII with an acid of formula XXXIII or a reactive derivative thereof.

(L) (Where a compound of formula I wherein A is =NO- is required) reaction of a compound of formula XXXIV with a compound of formula $NH_2$-O-$(Y)_m$-P.

(M) Formation of a group R1 by cyclisation of an appropriate precursor therefor.

For example a 2-aminothiazol-4-yl group may be formed by reacting a compound of formula XXXV (wherein R19 is a leaving group) with a compound of formula XXXVI (wherein R20 is amino or protected amino) followed by removal of the amino protecting group if present.

Certain cephalosporin compounds used as starting materials in the preparation of the compounds of the present invention are described in our published European Patent. Applications Nos. 127992 and 164944, or may be prepared by methods analogous to those described in the said applications.

In a further aspect the invention provides, as starting materials for the manufacture of antibacterial compounds of formula I, the following novel compounds as defined above:

1. A compound of formula XXV:
2. A compound of formula XXVIII;
3. A compound of formula XXXV.

When reference is made to protecting groups being present at any position in the compounds described herein such protecting groups may in general be chosen from any of the groups described in the literature or known to the skilled chemist as appropriate for the protection of the group in question, and may be introduced by conventional methods.

Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower' signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned is of course within the scope of the invention.

A carboxyl protecting group may be the residue of an ester-forming aliphatic or araliphatic alcohol or of an ester-forming phenol silanol or stannanol (the said alcohol, phenol, silanol or stannanol preferably containing 1-20 carbon atoms). Examples of carboxyl protecting groups include straight or branched chain (1-12C)alkyl groups (eg isopropyl, t-butyl); halo lower alkyl groups (eg 2-iodoethyl, 2,2,2-trichloroethyl); lower alkoxy lower alkyl groups (eg methoxymethyl, ethoxymethyl, isobutoxymethyl); lower aliphatic acyloxy lower alkyl groups, (eg acetoxymethyl, propionyloxymethyl, butyryloxymethyl, pivaloyloxymethyl); lower alkoxycarbonyloxy lower alkyl groups (eg 1-methoxycarbonyloxyethyl, 1-ethoxycarbonyloxyethyl); aryl lower alkyl groups (eg p-methoxybenzyl, o-nitrobenzyl, p-nitrobenzyl, benzhydryl and phthalidyl); tri(lower alkyl)silyl groups (eg trimethylsilyl and t-butyldimethylsilyl); tri(lower alkyl)silyl lower alkyl groups (eg trimethylsilylethyl); and (2-6C)alkenyl groups (eg allyl and vinylethyl).

Methods particularly appropriate for the removal of carboxyl protecting groups include for example acid-, base-metal- or enzymically-catalysed hydrolysis.

Examples of hydroxyl protecting groups include lower alkoxycarbonyl groups (eg t-butoxycarbonyl); halo lower alkoxycarbonyl groups (eg 2-iodoethoxycarbonyl, 2,2,2-trichloroethoxycarbonyl); aryl lower alkoxycarbonyl groups (eg benzoyloxycarbonyl, p-methoxybenzyloxycarbonyl, o-nitrobenzyloxycarbonyl, p-nitrobenzyloxycarbonyl); tri lower alkylsilyl (eg trimethylsilyl, t-butyldimethylsilyl) and aryl lower alkyl (eg benzyl) groups.

A (1-4C)alkanoyl group (eg acetyl) may also be regarded as a hydroxyl protecting group in the context of the compounds of the invention in that compounds of formula I in which W and/or Z represent (1-4C)-alkanoyloxy (eg acetoxy) groups (which are in themselves compounds of the invention) may readily be converted by conventional means into the corresponding hydroxy compounds.

Examples of amino protecting groups include formyl, aralkyl groups (eg benzyl and substituted benzyl, eg p-methoxybenzyl, nitrobenzyl and 2,4-dimethoxybenzyl, and triphenylmethyl); di-p-anisylmethyl and furylmethyl groups; acyl (eg alkoxycarbonyl and aralkoxycarbonyl eg t-butoxycarbonyl and benzyloxycarbonyl); trialkylsilyl (eg trimethylsilyl and t-butyldimethylsilyl); alkylidene (eg methylidine); benzylidene and

substituted benzylidene groups; and the phthalimido group.

As noted above the cephalosporin derivatives of the invention have antibacterial properties. Thus they may be useful antibacterial agents, having a broad spectrum of activity in vitro against standard laboratory microorganisms, both Gram-negative and Gram-positive, which are used to screen for activity against pathogenic bacteria. The antibacterial spectrum and potency of a particular compound may be determined in a standard test system. The compounds have particularly high activity against strains of Pseudomonas aeruginosa.

The antibacterial properties of compounds of the invention may also be demonstrated in vivo in conventional mouse protection tests.

Cephalosporin derivatives have generally been found to be relatively non-toxic to warm-blooded animals, and this generalisation holds true for the compounds of the present invention. A number of compounds were administered to mice at doses in excess of those required to afford protection against bacterial infections, and no overt toxic symptoms or side effects attributable to the administered compounds were noted.

According to a further feature of the invention there is provided a pharmaceutical composition which comprises an antibacterially active cephalosporin derivative of the invention in association with a pharmaceutically-acceptable diluent or carrier.

The pharmaceutical composition of the invention may, for example, be in a form suitable for oral, rectal or parenteral administration, for which purposes it may be formulated by means known to the art into the form of, for example, tablets, capsules, aqueous or oily solutions or suspensions, emulsions, dispersible powders, suppositories and sterile injectable aqueous or oily solutions or suspensions.

In addition to the cephalosporin derivative of the formula I the pharmaceutical composition of the invention may also contain, or be co-administered with, one or more known drugs selected from other clinically useful antibacterial agents (for example other beta-lactams or aminoglycosides), inhibitors of beta-lactamase (for example clavulanic acid), renal tubular blocking agents (e.g. probenicid) and inhibitors of metabolising enzymes (for example inhibitors of peptidases, for example Z-2-acylamino-3-substituted propenoates).

A preferred pharmaceutical composition of the invention is one suitable for intravenous, subcutaneous or intramuscular injection, for example a sterile injectable containing between 1 and 10% w/w of the cephalosporin derivative, or one suitable for oral administration in unit dosage form, for example a tablet or capsule which contains between 100 mg. and 1 g. of the cephalosporin derivative.

The pharmaceutical composition of the invention will normally be administered to man in order to combat infections caused by bacteria, in the same general manner as that employed for cephalothin, cefoxitin, cephradine, ceftazidine and other known clinically used cephalosporin derivatives, due allowance being made in terms of dose levels for the potency of the cephalosporin derivative of the present invention relative to the known clinically used cephalosporins. Thus each patient will receive a daily intravenous, subcutaneous or intramuscular dose of 0.1 to 50 g., and preferably 0.5 to 10 g., of the cephalosporin derivative, the composition being administered 1 to 4 times per day. The intravenous, subcutaneous and intramuscular dose may be given by means of a bolus injection. Alternatively the intravenous dose may be given by continuous infusion over a period of time. Alternatively each patient will receive a daily oral dose which is approximately equivalent to the daily parenteral dose. Thus a preferred daily oral dose is 0.5 to 10 g. of the cephalosporin derivative, the composition being administered 1 to 4 times per day.

The invention is illustrated by, but not limited to, the following Examples, in which the following abbreviations are used:

AcOH = acetic acid
DMF = dimethylformamide
DMSO = dimethylsulphoxide
EtOAc = ethyl acetate
EtOH = ethanol
HPLC = high performance liquid chromatography
MeOH = methanol
NMR = nuclear magnetic resonance spectroscopy
TEA = triethylamine
TFA = trifluoroacetic acid

The NMR spectra are taken at 90MHz and are quoted in terms of delta values in parts per million (ppm) with reference to tetramethylsilane (delta = 0).

In the quotation of NMR data s = singlet, d = doublet, t = triplet, q = quartet, m = mutiplet, br = broad.

Reference may be made to our published European Patent Applications Nos. 127992 and 164944 for descriptions of methods suitable for the preparation of any cephalosporin starting materials used in the following Examples and not otherwise known in the chemical literature.

## Example 1

7-[2-(2-Aminothiazol-4-yl)-2-[(Z)-3,4-diacetoxybenzoyl-oxyimino]acetamido]-3-[1-methyl-4-pyridinioamino-methyl]ceph-3-em-4-carboxylic acid

Diphenylmethyl 7-[2-(2-triphenylmethyl-aminothiazol-4-yl)-2-((Z)-hydroxyimino)acetamido]-3-[1-methyl-4-pyridinioaminomethyl]ceph-3-em-4-carboxylate (780 mg 0.72mmole) was dissolved in anhydrous dichloromethane (30 ml). To this solution at 0°C was added potassium carbonate (118 mg, 0.86 mmole) followed by 3,4-diacetoxybenzoyl chloride (220 mg, 0.86 mmole) in dichloromethane. After half an hour at 0°C the mineral salts were filtered off, and the organic phase washed with water, dried over magnesium sulphate and evaporated. 630 mg of diphenylmethyl 7-[2-(2-triphenylmethylaminothiazol-4-yl)-2-((Z)-3,4-diacetoxy-benzoyloxyimino)acetamido]-3-[1-methyl-4-pyridinioaminomethyl]ceph-3-em-4-carboxylate was obtained.

This product (690 mg, 0.5 mmole) in dichloromethane (4 ml), anisole (0.4 ml), TFA (0.8 ml) and water (2 drops) was stirred at room temperature for 1.5 h. The solution was precipitated in ether. The residue was purified by HPLC using HP 20 SS resin to obtain 172 mg of the title compound NMR-(DMSO$d_6$/AcOH$d_4$/TFA) 2.3 (s,6H); 3.5 (d,2H); 3.9 (s,3H); 4.3 (d,2H); 5.2(d,1H); 5.9 (d,1H); 7.9 and 8.0 (2d, 4H); 7.32(s,1H).

Diphenylmethyl 7-[2-(2-triphenylmethylaminothiazol-4-yl)-2-((Z)-hydroxyimino)acetamido]-3-[1-methyl-4-pyridinioaminomethyl]ceph-3-em-4-carboxylate used as starting material in the above Example was prepared as follows.

(a) To a stirred solution of 7-amino-3-[1-methyl-4-pyridinioaminomethyl]ceph-3-em-4-carboxylic acid trifluoroacetate (2.08 g, 4 mmole) and p-toluenesulphonic acid (760 mg, 4 mmole) in MeOH (10 ml) and acetonitrile (10 ml) was added diphenyl-diazomethane (2.5 g, 13 mmole) at room temperature. After stirring at room temperature the solvents were evaporated, the residue dissolved in MeOH and precipitated in ether. The precipitate was filtered, washed with ether, and dried over $P_2O_5$ under vacuum to yield 2.4 g of diphenylmethyl 7-amino-3-[1-methyl-4-pyridinioaminomethyl]ceph-3-em-4-carboxylate NMR (DMSO$d_6$/$CD_3COOD$/TFA) 2.3 (s,3H); 3.6 (s,2H), 3.9 (s,3H); 4.3 (s,2H); 5.3 (s,2H); 7.1(s,1H); 6.7-8.4 (m,18H).

(b) To a stirred solution of the product of (a) above (1.8 g, 2.4 mmole) in anhydrous dichloromethane (16 ml) with one equivalent of TEA (0.336 ml) at -15°C was added the reactive derivative of 2-[2-triphenylmethylaminothiazol-4-yl]-2-[(Z)-1-methoxy-1-methyl-ethoxyimino]acetic acid and the reaction mixture stirred at -15°C for 30 min. AcOH (0.1 ml) was then added and the product purified by low temperature silica gel chromatography eluting with dichloromethane/MeOH. 1.14 g of diphenylmethyl 7-[2-(2-triphenylmethylamino-thiazol-4-yl)-2-((Z)-1-methoxy-1-methylethoxyimino)-acetamido]-3-[1-methyl-4-pyridinioaminomethyl]ceph-3-em-4-carboxylate NMR($CDCl_3$/$D_2O$) 1.5 (s,6H); 2.3 (s,3H); 3.2 (s,3H); 3.6 (s,2H); 3.8 (s,3H); 4.4 (s,2H); 5.0 (d,1H); 6.0 (d,1H); 6.8 (s,1H); 7.0 (s, 1H); 7.1 and 7.8 (2d,4H); 6.6-8.0 (m, 29H).

(The reactive derivative of 2-[2-triphenyl-methylaminothiazol-4-yl]-2-[(Z)-1-methoxy-1-methyl-ethoxyimino]-acetic acid used in the above reaction was the compound prepared by reacting DMF (0.192 ml,2.4 mmole) with oxalyl chloride (0.208 ml, 2.4 mmole) in dichloromethane at 0°C to form a Vilsmeier salt which was then reacted with the acid in dichloromethane at 0°C in the presence of one equivalent of TEA for 30 min.)

(c) The product of (b) above was dissolved in acetone and cooled to 0°C. 1N HCl (1.4 ml) was added dropwise. The solution was stirred at room temperature for 6 h, diluted with 80 ml dichloromethane, washed with a saturated solution of NaCl and stirred over $MgSO_4$ to obtain 815 mg of the desired compound diphenylmethyl 7-[2-(2-triphenylmethylaminothiazol-4-yl)-2-((Z)-hydroxyimino)acetamido]-3-[1-methyl-4-pyridinioamino-methyl]ceph-3-em-4-carboxylate NMR($CDCl_3$/TFAd) 2.3 (s,3H); 3.6 (s,2H); 4.0 (s,3H); 4.3 (s,2H); 5.2 (d,1H); 5.8 (d,1H); 7.06 (s,1H), 7.0-8.4 (m,34H).

## Example 2

7-[2-(2-Aminothiazol-4-yl)-2-((Z)-3,4-diacetoxybenzoyloxyimino)acetamido]-3-[N-ethyl-N-(1-methyl-4-pyridinio)-aminomethyl]ceph-3-em-4-carboxylic acid

To 7-[2-(2-aminothiazol-4-yl)-2-((Z)-hydroxyimino)-acetamido]-3-[N-ethyl-N-(1-methyl-4-pyridinio)-aminomethyl]ceph-3-em-4-carboxylic acid (108 mg) in 3 ml AcOH at 40°C was added 40 mg of 3.4-diacetoxybenzoyl chloride. The mixture was stirred at 40°C for half an hour. After purification by chromatography over HP 20 SS resin 50 mg of the title compound was obtained NMR-(DMSO$d_6$.AcOHd/TFA) 1.1 (t,3H); 2.3 (s,6H); 3.2 and 3.5 (2d,2H); 3.6 (q,2H); 3.9 (s,3H); 4.6 (s,2H); 5.2 (d,1H); 5.9 (d, 1H); 7.3 (s, 1H); 7.1 and 8.2 (2d,4H); 7.4 (d,1H); 7.9 (s,1H); 7.9 (d,1H).

7-[2-(2-aminothiazol-4-yl)-2-((Z)-hydroxy-imino)acetamido]-3-[N-ethyl-N-(1-methyl-4-pyridinio)-aminomethyl]ceph-3-em-4-carboxylic acid used as starting material in the above reaction was prepared as follows.

(a) To a stirred solution of 7-amino-3-[N-ethyl-N-(1-methyl-4-pyridinioaminomethyl]ceph-3-em-4-carboxylic acid (1.07g, 2.24 mmole) and p-toluenesulphonic acid (855mg, 4.5 mmole) in MeOH (20ml) was added diphenyldiazomethane (1.5g, 7.7 mmole) at 0°C with stirring. After 1h the solution was precipitated in ether and the solid recovered and dried to obtain 2.28 g of diphenylmethyl 7-amino-3-[N-ethyl-N-(1-methyl-4-pyridinio)aminomethyl]ceph-3-em-4-carboxylate. NMR (DMSO$d_6$/$CD_3COOD$/TFAd) 1.0(t,3H); 2.3 (s,3H); 3.5 (s,2H); 3.2-3.6 (m,2H); 3.9 (s,3H); 4.65 (s,2H); 5.3 (s, 2H); 7.0 (s,1H); 7.12-7.56 (2d,4H); 7.4-8.2 (2d,4H); 7.0-7.6 (m,10H);

(b) To a stirred solution of the product of (a) above (2.28g 2.24 mmole) in 16ml of anhydrous dichloromethane with TEA (0.470ml, 3.36 mmole) at -30°C was added the reactive derivative of 2-[2-triphenylmethylaminothiazol-4-yl]-2-[(Z)-(1-methoxy-1-methylethoxyimino)]acetic acid (prepared by reaction of the acid with the vilsmeier salt as described in Example 1 (b)) and the reaction mixture stirred at 30°C for half an hour. A few drops of AcOH were added and the mixture washed with 0.5N HCl (40ml). The organic phase was chromatographed on silica at -20°C with dichloromethane/MeOH to obtain 550mg of diphenylmethyl 7-[2-(2-triphenylmethylaminothiazol-4-yl)-2((Z)-1-methoxy-1-methylethoxyimino)acetamido]-3-[N-ethyl-N-(1-methyl-4-pyridinio)aminomethyl]ceph-3-em-4-carboxylate.

(c) To a solution of the product of (b) above (480mg, 0.37 mmole) in acetone (4ml) was added 1.2ml of 1N HCl at room temperature with stirring. The mixture was stirred for 1 1/4h and the solvents were then evaporated. The mixture was dried under vacuum over $P_2O_5$ to yield 470mg of diphenylmethyl 7-[2-(2-triphenylmethylaminothiazol-4-yl)-2-((Z)-hydroxy-imino)acetamido]-3-(N-ethyl-N-(1-methyl-4-pyridinio)-aminomethyl]ceph-3-em-4-carboxylate. NMR(DMSO$d_6$/$CD_3COOD$/TFA) 1.0 (t,3H); 2.3 (s,3H); 3.0-3.6 (m,2H); 3.4 (s,2H); 3.9 (s,3H); 4.5 (s,2H); 5.2 (d,1H); 5.8 (d,1H); 6.8 (s,1H); 6.9 (s,1H); 7.1 and 7.5 (2d,4H); 7.4 and 8.2 (2d, 4H); 6.9-7.6 (m,25H).

(d) To the product of (c) above (440mg, 0.37 mmole) in dichloromethane (10ml) was added (at 0°C with stirring) a solution of 3,4-diacetoxybenzoyl chloride (108mg, 0.42mmol) in dichloromethane (5ml). After stirring for 30 minutes a few drops of TFA were added. The solvent was evaporated and the residue (534 mg) treated with TFA (3ml) and anisole (0.5ml) at 0°C to room temperature for 45 min. The product was purified by chromatography in HP 20 SS to yield 181mg of the desired compound 7-[2-(2-aminothiazol-4-yl)-2-((Z)-hydroxyimino)acetamido]-3-[N-ethyl-N-(1-methyl-4-pyridinio)aminomethyl]ceph-3-em-4-carboxylic acid. NMR (DMSO$d_6$/$CH_3COOD$/TFAd) 1.1 (t,3H); 2.3 (s,3H); 3.2 and 3.5 (2d,2H); 3.6 (q,2H); 3.9 (s,3H); 4.6 (s,2H); 5.18 (d,1H); 5.8 (d,1H); 6.9 (s,1H); 7.1 and 8.2 (2d,4H); 7.1

## Example 3

7-[2-(2-Aminothiazol-4-yl)-2-((Z)-carboxy(3,4-diacetoxyphenyl)methyl)oxyimino)acetamido]-3-[1-methyl-4-pyridinioaminomethyl]ceph-3-em-4-carboxylic acid (R and S isomers).

To a solution of diphenyl 7-amino-3-[1-methyl-4-pyridinioaminomethyl]ceph-3-em-4-carboxylate (340mg) (as described in Example 1) in dichloromethane (10ml), at -40°C, was added triethylamine (1 equivalent) followed by 2-(2-triphenylmethylaminothiazol-4-yl)-2-((Z)-(t-butoxycarbonyl(3,4-diacetoxyphenyl)methyl)-oxyimino)acetyl chloride (one equivalent). The reaction mixture was allowed to warm to -20°C. during a period of one hour. Trifluoroacetic acid (20 drops) was added, the mixture was evaporated under reduced pressure and the resultant residue was taken up in ethyl acetate, washed with water, dried ($MgSO_4$) and evaporated to give a residue. This was treated with trifluoroacetic acid (20ml) and water (2ml) at room

temperature for 2 hours. The majority ( 80%) of the acid was evaporated and the residue was subjected to column chromatography on HP20SS using methanol:water (1% acetic acid) gradient elution. A mixture (42 mg) of the diastereoisomers was obtained; nmr (more hydrophilic isomer) 2.24(s,6H), 3.5(m,2H), 3.87(s,3H), 4.35(m,2H), 5.19(d,1H), 5.74(s,1H), 5.82(d,1H), 6.8-8.3(m,8H).

2-(2-Triphenylmethylaminothiazol-4-yl)-2-((Z)-(t-butoxycarbonyl(3,4-diacetoxyphenyl)methyl)oxyimino) acetyl chloride used as starting-material in the above reaction was prepared as follows:-

a) 3,4-Dihydroxyphenylacetic acid (8.4g) was suspended, with stirring, in acetic anhydride (13,6ml). Concentrated sulphuric acid was added, the mixture was cooled, stirred to give a solution, stirred at ambient temperature for a further 45 minutes and subjected to column chromatography on silica, using dichloromethane-ether mixtures (gradient elution) to give 3,4-diacetoxyphenylacetic acid (12.5g); NMR 2.26-(s,6H), 3.63(s,2H), 7.75(s,3H).

b) The product from a) above (9.1g) was suspended in thionyl chloride (26ml), and stirred under reflux for one hour. The mixture was cooled and evaporated to give 3,4-diacetoxyphenylacetyl chloride (9.7g) as a crystalline oil; NMR 2.28(s,6H), 4.12(s,2H), 7.17(s,3H).

c) The product from b) above (8.6g) was partially dissolved in carbon tetrachloride (35ml) with slight heating. N-Bromosuccinimide (7.1g) was added, with stirring, followed by p-toluenesulphonic acid (100mg). The mixture was heated at 80° C for 2 hours, filtered and evaporated to give α-bromo-3,4-diacetoxyphenyl acetyl chloride (11.2g) NMR 2.29(s,6H), 5.61(s,1H), 7.19-7.43(m,3H).

d) The product from c) above (11.2g) was dissolved in anhydrous dichloromethane (120ml), t-butanol (3.45ml) and subsequently pyridine (3ml) were added, with stirring, at 0° C. The mixture was allowed to warm to room temperature and was stirred for a further 2 1/2 hours. The solvents were evaporated under reduced pressure and the residue subjected to column chromatography on silica, using dichloromethane as eluent, to give t-butyl α-bromo-3,4-diacetoxyphenyl acetate (6.8g); NMR 1.44(s,9H), 2.28(s,6H), 5.19(s,1H), 7.13-7.43(m,3H).

e) The product from d) above (1.94) and N-hydroxyphthalimide (820mg) were dissolved in dimethylformamide (20ml). Triethylamine (700µl) was added and the mixture was stirred for 75 minutes at ambient temperature. The solvents were removed by evaporation, the residue was dissolved in dichloromethane and acetyl chloride (360µl) and triethylamine (700µl) were added. The mixture was stirred for 20 minutes and purified by column chromatography on silica using dichloromethane-ether gradient elution to give t-butyl (α-N-phthalimido-oxy)3,4-diacetoxyphenylacetate (1.07g); NMR 1.44(s,9H), 2.26(s,6H), 5.74(s,1H), 7.16-7.86-(m,7H).

f) The product from e) above (3.5g) was dissolved in dichloromethane (90ml) with stirring. Hydrazine hydrate (1.16ml) and methanol (8ml) were added to give a solution. The mixture was stirred at ambient temperature for 80 minutes, the solvents were evaporated and the residue was subjected to column chromatography on silica using dichloromethane-ether gradient elution to give t-butyl α-aminooxy-3,4-dihydroxy phenylacetate (1.18g); NMR 1.39(s,3H), 5.33(s,1H), 6.72-6.85(m,3H).

g) The product from f) above (1.14g) and 2-(2-triphenylmethylaminothiazol-4-yl) oxalic acid (1.9g) were stirred in dichloromethane (40ml) for 90 minutes, and then evaporated under reduced pressure to give 2-(2-triphenylmethylaminothiazol-4-yl)-2-((Z)-(t-butoxycarbonyl(3,4-dihydroxyphenyl)methyl)oxyimino) acetic acid (2.9g); NMR 1.32(s,9H), 5.43(s,1H), 6.69(s,1H), 7.25(s,15H).

h) To the product from g) above (2.9g) in dichloromethane (35ml), at 0° C, with stirring, was added acetyl chloride (628µl) followed by pyridine (710µl). After 30 minutes further acetyl chloride (628µl) and pyridine (710µl) were added. The solvents were evaporated under reduced pressure and the residue subjected to refrigerated column chromatography on silica using dichloromethaneether (95:5) as eluant to give 2-(2-triphenylmethylaminothiazol-4-yl)-2-((z)-(t-butoxycarbonyl(3,4-diacetoxyphenyl)methyl)oxyimino) acetic acid (3.2g); NMR 1.39(s,9H), 2.25(s,6H), 5.75(s,1H), 6.81(s,1H), 7.28(s,15H).

i) To the product from h) above (1.47g) in dichloromethane (45ml), at 0° C, was added phosphorous pentachloride (460mg) followed by triethylamine (306µl). After 40 minutes stirring at 0° C the solvents were evaporated, the residue was taken up in dichloromethane, filtered and evaporated to give the starting material for Example 3.

## Claims

1. A cephalosporin derivative of the formula I in which X is sulphur, oxygen, methylene or sulphinyl (R or S configuration);
R1 is 2-aminothiazol-4-yl or 2-aminooxazol-4-yl each optionally substituted in the 5-position by fluorine, chlorine or bromine, or R1 is 5-amino-isothiazol-3-yl, 5-amino-1,2,4-thiadiazol-3-yl, 3-aminopyrazol-5-yl, 3-aminopyrazol-4-yl, 2-aminopyrimidin-5-yl, 2-aminopyrid-6-yl, 4-aminopyrimidin-2-yl, 2-amino-1,3,4-thiadiazol-5-yl or 5-amino-1-methyl-1,2,4-triazol-3-yl;

R2 is hydrogen, methoxy or formamido;

R3 is hydrogen, (1-4C)alkyl, halo(1-4C)alkyl, hydroxy(1-4C)alkyl, (1-4C)alkoxy(1-4C)alkyl, carboxy (1-4C)-alkyl, amino)1-4C)alkyl, cyano(1-4C)alkyl, (1,-4C) alkanoylamino(1-4C)alkyl, (3-6C)alkenyl, phenyl(1-4C)alkyl or heteroaryl(1-4C)alkyl wherein heteroaryl is a 5- or 6- membered ring containing 1, 2 or 3 heteroatoms selected from nitrogen, oxygen and sulphur;

A is of the formula =NO-, =N-NH- or =CH-;

Y is -CO- or a group of formula

$$-(CH_2)_n-\underset{R^b}{\overset{R^a}{C}}-(CH_2)_{n'}-(Y')_p-$$

(wherein Ra and Rb are hydrogen or (1-3C)alkyl or together represent (3-7C)cycloalkyl or (1-8C)alkylidene. n and n′ are independently 0-3, p is 0 or 1 and Y′ is -CO-, or (when A is of the formula =NO-) Y′ may additionally be selected from -CONH-, -NHCO-, -C(=O)O-, -OC(=O)-, $-NHSO_2$, $-SO_2NH-$, -O-, -S- and $-NR^c-$ where $R^c$ is hydrogen, (1-4C)alkyl or (2-4C)alkenyl) the said group being optionally substituted by carboxy.

m is 0 or 1 such that when m=0 A is connected directly to ring P by a covalent bond;

P represents:

(i) a benzene ring, optionally fused to a further benzene ring (so forming a naphthyl group) or to a 5 or 6 membered heterocyclic aromatic group containing 1, 2 or 3 heteroatoms selected from nitrogen, oxygen and sulphur and said ring system substituted by groups W and Z which are ortho with respect to one another wherein W is hydroxy or an in vivo hydrolysable ester thereof and Z is hydroxy or an in vivo hydrolysable ester thereof, carboxy, sulpho, hydroxymethyl, $-NHSO_2CH_3$ or $-NHCONH_2$

(ii) a group of formula (II) or

(iii) a group of formula (III) and tautomers thereof wherein M represents oxygen or $NR^d$ wherein $R^d$ represents hydrogen or (1-4C)alkyl,

ring system P optionally being further substituted by (1-4C)alkyl, halogen, hydroxy, cyano, trifluoromethyl, nitro, amino, mono- or di-(1-4C)alkylamino, formyl, (1-4C)alkanoy, (1-4C)alkoxy, (1-4C)alkylthio, (1-4C)-alkanoyloxy, carbamoyl or mono- or di(1-4C)alkyl-carbamoyl;

Q represents a 5 to 10 membered mono- or bicyclic heterocyclic ring system bonded to NR3 via a carbon atom and containing, in addition to the positively charged nitrogen atom, 0 to 5 further heteroatoms selected from nitrogen, oxygen and sulphur and being optionally substituted:

on a carbon atom or atoms available for substitution by 1,2 or 3 groups R4 wherein R4 is halogen, (1-6C)-alkyl, carboxy, (2-6C)alkoxycarbonyl, (2-6C)alkoxycarbonyl(1-4C)alkyl, (1-6C)alkoxy, (1-6C)alkylthio, cyano, (1-4C) cyanoalkyl, amino, (1-6C)-alkylamino, (2-8C)dialkylamino, phenyl(1-4C)alkylamino, nitrophenyl(1-4C)-alkylamino, heteroaryl(1-4C)alkyl (wherein heteroaryl is as hereinbefore defined), (3-6C)alkenylamino, amino(1-6C)alkylamino, (1-6C)alkoxy(1-6C)alkylamino, hydroxy(1-6C)alkylamino, hydroxy, mercapto, carbamoyl, (2-6C) alkylcarbamoyl, (3-10C)dialkylcarbamoyl, phenylthio and heteroarylthio (wherein heteroaryl is hereinbefore defined) wherein when more than one group R4 is present these may be the same or different;

on an uncharged nitrogen atom available for substitution by a group R5 wherein R5 is (1-6C)alkyl, phenyl or benzyl;

R6 is hydrogen, (1-6C)alkyl (optionally substituted by carboxy, (1-6C)alkoxycarbonyl, carbamoyl, mono- or di-(1-4C)alkylcarbamoyl, hydroxy, (1-4C)alkoxy, amino, mono- or di-(1-4C)alkylamino, (1-4C)alkanoyl, benzoyl, cyano, carboxyaminocarbonyl, (1-6C)alkoxy-carbonylaminocarbonyl, (1-4C)alkoxy(2-4C)alkoxy or phenyl), (1-6C)alkoxy, phenyl(1-6C)alkoxy, amino, (1-6C)alkylamino, (3-6C)cycloalkyl, (3-6C)cycloalkenyl, cyano(3-6C)cycloalkenyl, (2-6C)alkenyl (optionally substituted by (1-6C)alkyl, halogen, cyano, carbamoyl, mono- or di-(1-4C)alkylcarbamoyl, piperidinocarbonyl or morpholinocarbonyl), 2-ureidoethyl, 2-thioureidoethyl, 2-(thioacetylamino)ethyl, sulphamoyl, 2-amino-2-carboxyethyl, acetylaminomethyl, phthalimidomethyl, 4-5-dihydroimidazol-2-yl-methyl, 3,4,5,6-tetrahydropyrimidin-2-ylmethyl, 2-(1,2,3,6-tetrahydro-2,6-dioxopurin-7-ylethyl, 2-hydroxyiminopropyl (syn or anti) [(1-4C)alkoxyimino]propyl (syn or anti) or phenyl,

or R6 is of the formula $-(CH_2)_2$-NR7R8R9 in which R7, R8 and R9 are (1-4C)alkyl,

or R6 is of the formula $-(CH_2)_s$-R10 in which s is 0-2 and R10 is pyridine, pyridazine, pyrimidine, pyrazine, 1,2,5,6-dihydro-5,6-dioxo-1,2,4-triazine, 2-[(1-4C)alkyl]-1,2,5,6-dihydro-5,6-dioxo-1,2,4-triazine, 1-[(1-4C)-alkyl]tetrazole, furan, thiophene, pyrrole, 1-[(1-4C)alkyl]pyrrole, oxazole, thiazole, imidazole, 1-[1-4C)alkyl]-imidazole, isoxazole, isothiazole, pyrazole, 1,2,3-thiadiazole, 1-[(1-4C)alkyl]pyrazole, benzfuran, benzthiophene, indole, oxindole, 1-[(1-4C)alkyl]indole, benzoxazole, benzthiazole, benzimidazole, 1-[(1-4C)alkyl]-benzimidazole, or 3,4-dihydro-4-oxo-2H-benzo[e]oxazine (each of these ring systems being linked to $(CH_2)_s$ through carbon and each ring system being optionally substituted by halogen, (1-6C)alkyl, (1-4C)haloalkyl, (3-6C)cycloalkyl, (3-6C)cycloalkyl(1-4C)alkyl, (2-6C)alkenyl, carboxy, (2-6C)alkoxycarbonyl, (1-6C)alkoxy, cyano, (2-6C)cyanoalkenyl, carbamoyl, mono-or di-(1-4C)-alkylcarbamoyl, (1-4C)alkanoylamino, guanidino, hydroxy, nitro or amino),

or R6 is 2-guanidino-thiazol-4-ylmethyl, hydroxybenzoylmethyl, 2-thenyl, 2-imidazolylmethyl or cinnamyl (each optionally substituted by halogen, (1-6C)alkyl, hydroxy, (1-4C)alkoxy, carboxy, (2-6C)-alkoxycarbonyl, nitro or carbamoyl),

or R6 is $-(CH_2)_t$NHCOR11 or $-(CH_2)_tS(O)_u$-R11 in which t is 1-6, u is 0, 1 or 2 and R11 is (1-6C)alkyl or (1-6C)alkoxy,

or R6 is of the formula $(CH_2)_2$N=CR12NR13R14 or $-(CH_2)_v$C(=NR12)NR13R14 or a tautomer thereof in which v is 1-6 and R12, R13, R14 are hydrogen or (1-4C)alkyl,

(wherein when R6 is or contains a phenyl group, the phenyl group is optionally substituted by 1 or 2 groups selected from halogen, (1-6C)alkyl, (1-6C)alkoxy, amino, carboxy, nitro, carbamoyl, cyano, trifluoromethyl, aminomethyl, (1-4C)alkanoyl, (1-4C)alkanoylamino, halo(1-4C)alkyl, (2-6C)alkoxycarbonyl, mono- or di-(1-4C)alkylcarbamoyl, mesyl, vinyl, sulpho, sulphamoyl or mono- or di(1-4C)alkylsulphamoyl);

or, where the positively charged nitrogen atom in ring Q occurs at the junction between fused rings, R6 is absent;

and the N-oxides thereof where chemically possible;

and the salts formed with acids and bases which afford pharmaceutically acceptable anions and cations respectively.

2. A compound according to claim 1 wherein X is sulphur and $R^2$ is hydrogen.

3. A compound according to either claim 1 or claim 2 wherein $R^1$ is 2-aminothiazol-4-yl and A is a group =NO-.

4. A compound according to any one of claims 1 to 3 wherein -(Y)m- is -CO- or $C_{1-4}$alkylene optionally substituted by carboxy.

5. A compound according to any one of claims 1 to 4 wherein P is a benzene ring wherein both W and Z are hydroxy and said ring is optionally substituted by halo.

6. A compound according to any one of claims 1 to 5 wherein $R^3$ is hydrogen or $C_{1-4}$alkyl.

7. A compound according to claim 1 which is:-7-[2-(2-aminothiazol-4-yl)-2-[(Z)-3,4-diacetoxybenzoyl-oxyimino]acetamido-3-[1-methyl-4-pyridiniominomethyl]ceph-3-em-4- carboxylic acid.

7-[2-(2-aminothiazol-4-yl)-2-((Z)-3,4-diacetoxybenzoyl-oxyimino)-acetamido]-3-[N-ethyl-N-(1-methyl-4-pyridinio)-aminomethyl]ceph-3-em-4-carboxylic acid or

7-[2-(2-aminothiazol-4-yl)-2-((Z)-carboxy(3,4-diacetoxyphenyl)methyl)-oxyimino)acetamido]-3-[1-methyl-4-pyridinioaminomethyl]ceph-3-em-4-carboxylic acid (R and S isomers).

8. A pharmaceutical composition which comprises a compound according to any one of claims 1 to 7 and a pharmaceutically acceptable carrier.

9. A process for preparing a compound as defined in claim 1 which comprises:

a) reacting a compound of the formula XXV wherein L is a leaving group with a nucleophilic compound serving to form the desired group $-NR^3$-Q-$R^6$; or

b) reacting a compound of the formula XXVIII with a compound of the formula XXIX wherein $R^{18}$ is a leaving group; or

c) reacting a compound of the formula XXX with a compound of the formula XXXI wherein J and K are such that reaction takes place to form the link -(Y)m- between A and the ring P; or

d) deprotecting, if it is desired to form a compound wherein W and Z are hydroxy groups, a corresponding compound wherein one or both of W and Z are protected hydroxy groups; or

e) esterifying, if it is desired to form a compound wherein W and Z are in vivo hydrolysable ester groups, a corresponding compound wherein W and Z are hydroxy groups; or

f) deprotecting, if it is desired to form carboxy, a corresponding compound containing a protected carboxy group; or

g) deprotecting, if it is desired to form amino, a corresponding compound containing a protected amino group; or

h) for compounds wherein X is sulphur or sulphinyl, converting sulphur to sulphinyl or vice versa: or

i) reacting a compound of the formula XXXII with an acid of the formula XXXIII or a reactive derivative thereof; or

j) for preparing a compound wherein A is =NO-, reacting a compound of the formula XXXIV with a compound of the formula $NH_2$-O-(Y)m-P or

k) forming a group $R^1$ by cyclizing an appropriate precursor: and thereafter, if desired, forming a pharmaceutically acceptable salt.

10. A compound of the formula XXV, XXVIII or XXXV.

Claims for the following contracting States: GR, ES.

1. A process for preparing a cephalosporin derivative of the formula I in which X is sulphur, oxygen, methylene or sulphinyl (R or S configuration);
R1 is 2-aminothiazol-4-yl or 2-aminooxazol-4-yl each optionally substituted in the 5-position by fluorine, chlorine or bromine, or R1 is 5-amino-isothiazol-3-yl, 5-amino-1,2,4-thiadiazol-3-yl, 3-aminopyrazol-5-yl, 3-aminopyrazol-4-yl, 2-aminopyrimidin-5-yl, 2-aminopyrid-6-yl, 4-aminopyrimidin-2-yl, 2-amino-1,3,4-thiadiazol-5-yl or 5-amino-1-methyl-1,2,4-triazol-3-yl;
R2 is hydrogen, methoxy or formamido;
R3 is hydrogen, (1-4C)alkyl, halo(1-4C)alkyl, hydroxy(1-4C)alkyl, (1-4C)alkoxy(1-4C)alkyl, carboxy (1-4C)-alky, amino(1-4C)alkyl, cyano)1-4C)alkyl, (1-4C) alkanoylamino(1-4C)alkyl, (3-6C)alkenyl, phenyl(1-4C)alkyl or heteroaryl(1-4C)alkyl wherein heteroaryl is a 5- or 6- membered ring containing 1, 2 or 3 heteroatoms selected from nitrogen, oxygen and sulphur;
A is of the formula =NO-, =N-NH- or =CH-;
Y is -CO- or a group of formula

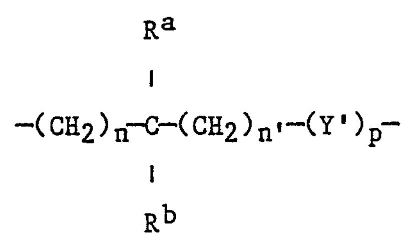

(wherein Ra and Rb are hydrogen or (1-3C)alkyl or together represent (3-7C)cycloalkyl or (1-8C)alkylidene, n and n′ are independently 0-3, p is 0 or 1 and Y′ is -CO-, or (when A is of the formula =NO-) Y′ may additionally be selected from -CONH-, -NHCO-, -C(=O)O-, -OC(=O)-, -$NHSO_2$, -$SO_2$NH-, -O-, -S- and -$NR^c$- where $R^c$ is hydrogen, (1-4C)alkyl or (2-4C)alkenyl) the said group being optionally substituted by carboxy.
m is 0 or 1 such that when m=0 A is connected directly to ring P by a covalent bond;
P represents:
(i) a benzene ring, optionally fused to a further benzene ring (so forming a naphthyl group) or to a 5 or 6 membered heterocyclic aromatic group containing 1, 2 or 3 heteroatoms selected from nitrogen, oxygen and sulphur and said ring system substituted by groups W and Z which are ortho with respect to one another wherein W is hydroxy or an in vivo hydrolysable ester thereof and Z is hydroxy or an in vivo hydrolysable ester thereof, carboxy, sulpho, hydroxymethyl, -$NHSO_2CH_3$ or -$NHCONH_2$
(ii) a group of formula (II) or
(iii) a group of formula (III) and tautomers thereof wherein M represents oxygen or $NR^d$ represents hydrogen or (1-4C)alkyl,
ring system P optionally being further substituted by (1-4C)alkyl, halogen, hydroxy, cyano, trifluoromethyl, nitro, amino, mono- or di-(1-4C)alkylamino, formyl, (1-4C)alkanoyl, (1-4C)alkoxy, (1-4C)alkylthio, (1-4C)-alkanoyloxy, carbamoyl or mono- or di(1-4C)alkyl-carbamoyl;
Q represents a 5 to 10 membered mono- or bicyclic heterocyclic ring system bonded to NR3 via a carbon atom and containing, in addition to the positively charged nitrogen atom, 0 to 5 further heteroatoms selected from nitrogen, oxygen and sulphur and being optionally substituted:

on a carbon atom or atoms available for substitution by 1,2 or 3 groups R4 wherein R4 is halogen, (1-6C)-alkyl, carboxy, (2-6C)alkoxycarbonyl, (2-6C)alkoxycarbonyl(1-4C)alkyl, (1-6C)alkoxy, (1-6C)alkylthio, cyano, (1-4C) cyanoalkyl, amino, (1-6C)-alkylamino, (2-8C)dialkylamino, phenyl(1-4C)alkylamino, nitrophenyl(1-4C)-alkylamino, heteroaryl(1-4C)alkyl (wherein heteroaryl is as hereinbefore defined), (3-6C)alkenylamino, amino(1-6C)alkylamino, (1-6C)alkoxy(1-6C)alkylamino, hydroxy(1-6C)alkylamino, hydroxy, mercapto, carbamoyl, (2-6C) alkylcarbamoyl, (3-10C)dialkylcarbamoyl, phenylthio and heteroarylthio (wherein heteroaryl is hereinbefore defined) wherein when more than one group R4 is present these may be the same or different;

on an uncharged nitrogen atom available for substitution by a group R5 wherein R5 is (1-6C)alkyl, phenyl or benzyl;

R6 is hydrogen, (1-6C)alkyl (optionally substituted by carboxy, (1-6C)alkoxycarbonyl, carbamoyl, mono- or di-(1-4C)alkylcarbamoyl, hydroxy, (1-4C)alkoxy, amino, mono- or di-(1-4C)alkylamino, (1-4C)alkanoyl, benzoyl, cyano, carboxyaminocarbonyl, (1-6C)alkoxy-carbonylaminocarbonyl, (1-4C)alkoxy(2-4C)alkoxy or phenyl), (1-6C)alkoxy, phenyl(1-6C)alkoxy, amino, (1-6C)alkylamino, (3-6C)cycloalkyl, (3-6C)cycloalkenyl, cyano(3-6C)cycloalkenyl, (2-6C)alkenyl (optionally substituted by (1-6C)alkyl, halogen, cyano, carbamoyl, mono- or di-(1-4C)alkylcarbamoyl, piperidinocarbonyl or morpholinocarbonyl), 2-ureidoethyl, 2-thioureidoethyl, 2-(thioacetylamino)ethyl, sulphamoyl, 2-amino-2-carboxyethyl, acetylaminomethyl, phthalimidomethyl, 4-5-dihydroimidazol-2-yl-methyl, 3,4,5,6-tetrahydropyrimidin-2-ylmethyl, 2-(1,2,3,6-tetrahydro2,6-dioxopurin-7-ylethyl, 2-hydroxyiminopropyl (syn or anti) [(1-4C)alkoxyimino]propyl (syn or anti) or phenyl,

or R6 is of the formula $-(CH_2)_2$-NR7R8R9 in which R7, R8 and R9 are (1-4C)alkyl,

or R6 is of the formula $-(CH_2)_s$-R10 in which s is 0-2 and R10 is pyridine, pyridazine, pyrimidine, pyrazine, 1,2,5,6-dihydro-5,6-dioxo-1,2,4-triazine, 2-[(1-4C)alkyl]-1,2,5,6-dihydro-5,6-dioxo-1,2,4-triazine, 1-[(1-4C)-alkyl]tetrazole, furan, thiophene, pyrrole, 1-[(1-4C)alkyl]pyrrole, oxazole, thiazole, imidazole, 1-[1-4C)alkyl]-imidazole, isoxazole, isothiazole, pyrazole, 1,2,3-thiadiazole, 1-[(1-4C)alkyl]pyrazole, benzfuran, benzthiophene, indole, oxindole, 1-[(1-4C)alkyl]indole, benzoxazole, benzthiazole, benzimidazole, 1-[(1-4C)alkyl]-benzimidazole, or 3,4-dihydro-4-oxo-2H-benzo[e]oxazine (each of these ring systems being linked to $(CH_2)_s$ through carbon and each ring system being optionally substituted by halogen, (1-6C)alkyl, (1-4C)haloalkyl, (3-6C)cycloalkyl, (3-6C)cycloalkyl(1-4C)alkyl, (2-6C)alkenyl, carboxy, (2-6C)alkoxycarbonyl, (1- 6C)alkoxy, cyano, (2-6C)cyanoalkenyl, carbamoyl, mono-or di-(1-4C)-alkylcarbamoyl, (1-4C)alkanoylamino, guanidino, hydroxy, nitro or amino),

or R6 is 2-guanidino-thiazol-4-ylmethyl, hydroxybenzoylmethyl, 2-thenyl, 2-imidazolylmethyl or cinnamyl (each optionally substituted by halogen, (1-6C)alkyl, hydroxy, (1-4C)alkoxy, carboxy, (2-6C)-alkoxycarbonyl, nitro or carbamoyl),

or R6 is $-(CH_2)_t$NHCOR11 or $-(CH_2)_tS(O)_u$-R11 in which t is 1-6, u is 0, 1 or 2 and R11 is (1-6C)alkyl or (1-6C)alkoxy,

or R6 is of the formula $(CH_2)_2N=CR12NR13R14$ or $-(CH_2)_vC(=NR12)NR13R14$ or a tautomer thereof in which v is 1-6 and R12, R13, R14 are hydrogen or (1-4C)alkyl, (wherein when R6 is or contains a phenyl group, the phenyl group is optionally substituted by 1 or 2 groups selected from halogen, (1-6C)alkyl, (1-6C)alkoxy, amino, carboxy, nitro, carbamoyl, cyano, trifluoromethyl, aminomethyl, (1-4C)alkanoyl, (1-4C)-alkanoylamino, halo(1-4C)alkyl, (2-6C)alkoxycarbonyl, mono- or di-(1-4C)alkylcarbamoyl, mesyl, vinyl, sulpho, sulphamoyl or mono- or di(1-4C)alkylsulphamoyl);

or, where the positively charged nitrogen atom in ring Q occurs at the junction between fused rings, R6 is absent;

and the N-oxides thereof where chemically possible;

and the salts formed with acids and based which afford pharmaceutically acceptable anions and cations respectively:

which process comprises:

a) reacting a compound of the formula XXV wherein L is a leaving group with a nucleophilic compound serving to form the desired group $-NR^3-Q-R^6$; or

b) reacting a compound of the formula XXVIII with a compound of the formula XXIX wherein $R'^8$ is a leaving group; or

c) reacting a compound of the formula XXX with a compound of the formula XXXI wherein J and K are such that reaction takes place to form the link -(Y)m- between A and the ring P; or

d) deprotecting, if it is desired to form a compound wherein W and Z are hydroxy groups, a corresponding compound wherein one or both of W and Z are protected hydroxy groups; or

e) esterifying, if it is desired to form a compound wherein W and Z are in vivo hydrolysable ester groups, a corresponding compound wherein W and Z are hydroxy groups; or

f) deprotecting, if it is desired to form carboxy, a corresponding compound containing a protected carboxy group; or

g) deprotecting, if it is desired to form amino, a corresponding compound containing a protected amino group; or

h) for compounds wherein X is sulphur or sulphinyl, converting sulphur to sulphinyl or vice versa; or

i) reacting a compound of the formula XXXII with an acid of the formula XXXIII or a reactive derivative thereof; or

j) for preparing a compound wherein A is =NO-, reacting a compound of the formula XXXIV with a compound of the formula $NH_2$-O-(Y)m-P; or

k) forming a group $R^1$ by cyclizing an appropriate precursor: and thereafter, if desired, forming a pharmaceutically acceptable salt.

2. A process according to claim 1 for preparing a compound of the formula (I) wherein X is sulphur and $R^2$ is hydrogen.

3. A process according to either claim 1 or claim 2 for preparing a compound of the formula (1) wherein $R^1$ is 2-aminothiazol-4-yl and A is a group =NO-.

4. A process according to any one of claims 1 to 3 for preparing a compound of the formula (1) wherein -(Y)m- is -CO- or $C_{1-4}$alkylene optionally substituted by carboxy.

5. A process according to any one of claims 1 to 4 for preparing a compound of the formula (I) wherein P is a benzene ring wherein both W and Z are hydroxy and said ring is optionally substituted by halo.

6. A process according to any one of claims 1 to 5 for preparing a compound of the formula (I) wherein $R^3$ is hydrogen or $C_{1-4}$alkyl.

7. A process according to claim 1 for preparing a compound which is:-
7-[2-(2-aminothiazol-4-yl)-2-[(Z)-3,4-diacetoxybenzoyloxyimino]acetamido]-3-[1-methyl-4-pyridinioaminomethyl]ceph-3-em-4-carboxylic acid,
7-[2-(2-aminothiazol-4-yl)-2-((Z)-3,4-diacetoxybenzoyl-oxyimino)-acetamido]-3-[N-ethyl-N-(1-methyl-4-pyridinio)-aminomethyl]ceph-3-em-4-carboxylic acid or
7-[2-(2-aminothiazol-4-yl)-2-((Z)-carboxy(3,4-diacetoxyphenyl)-methyl)oxyimino)acetamido]-3-[1-methyl-4-pyridinioaminomethyl]ceph-3-em-4-carboxylic acid (R and S isomers).

8. A process for preparing pharmaceutical composition which comprises bringing into association a compound of the formula (1) and a pharmaceutically acceptable carrier.

9. A compound of the formula XXV, XXVIII or XXXV.

FORMULAE IN CLAIMS

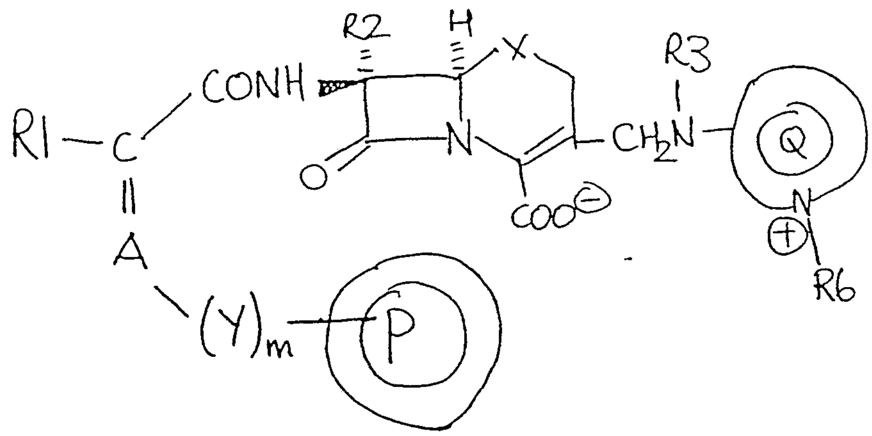

(I)

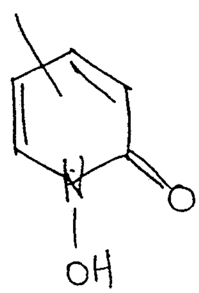

(II)

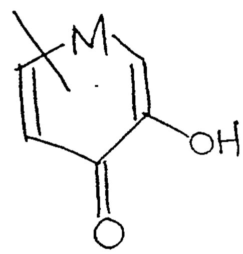

(III)

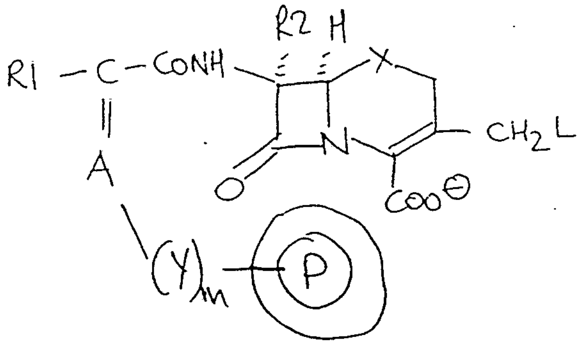

(XXV)

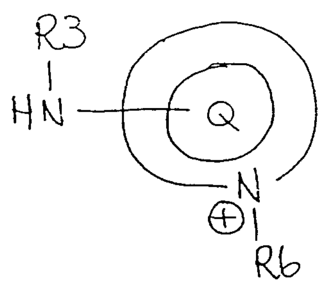

(XXVI)

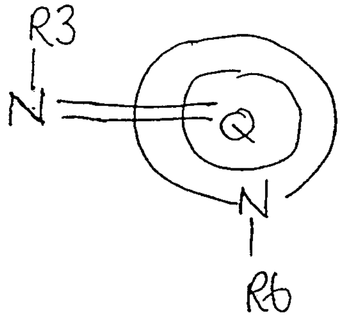

(XXVII)

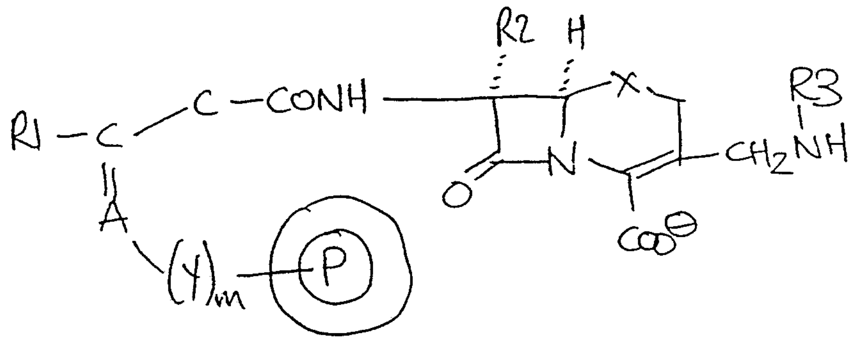

(XXVIII)

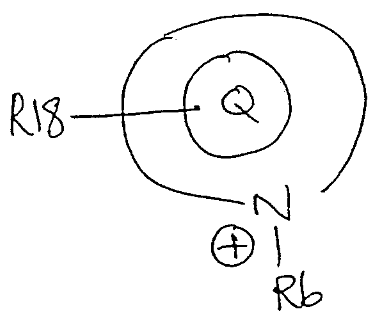

(XXIX)

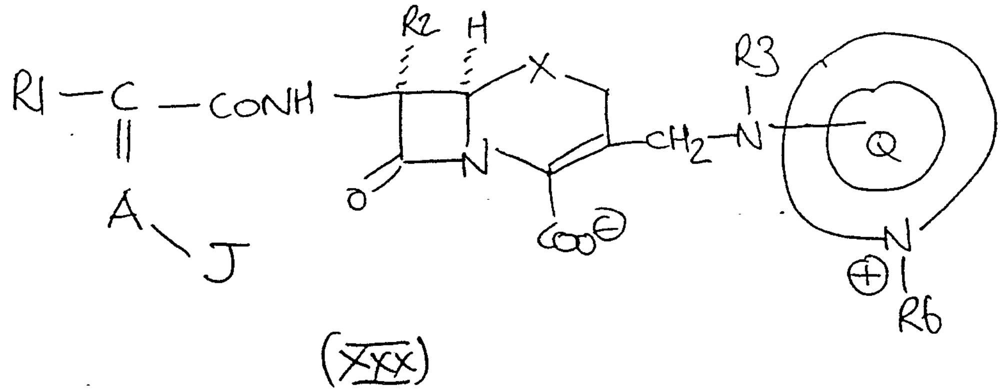

(XXX)

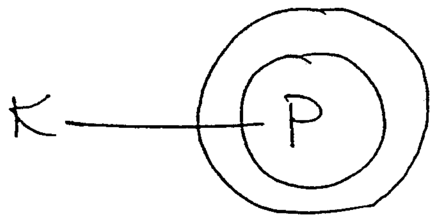

(XXXI)

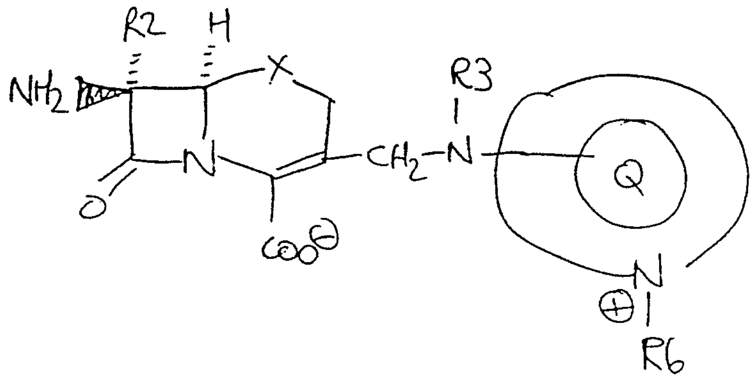

(XXXII)

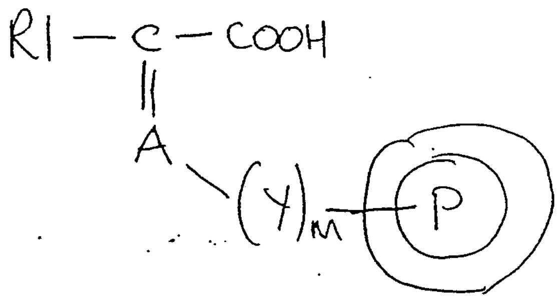

(XXXIII)

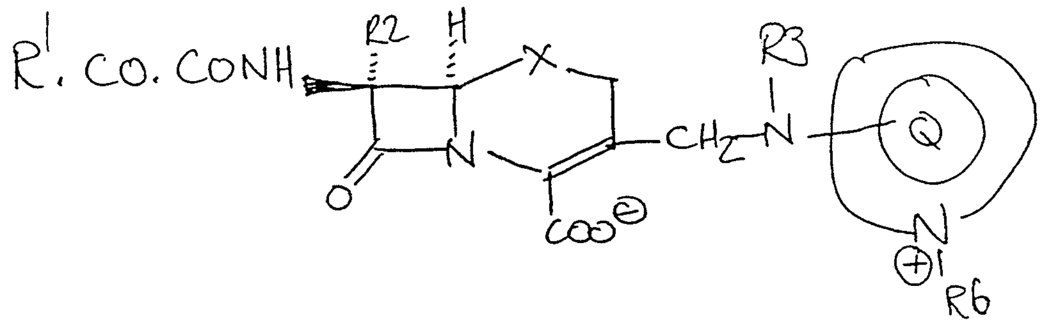

(XXXIV)

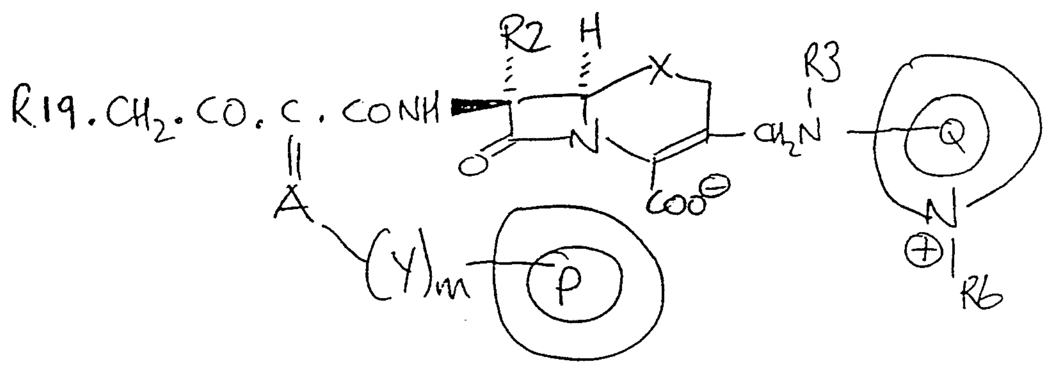

(XXXV)

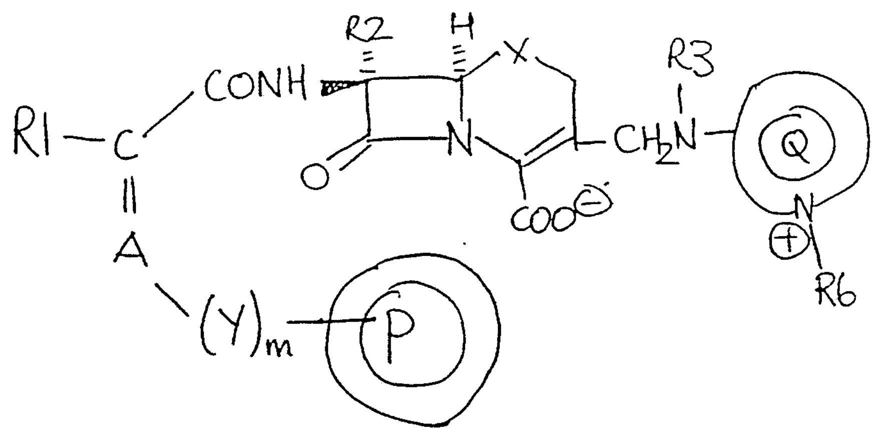

(I)

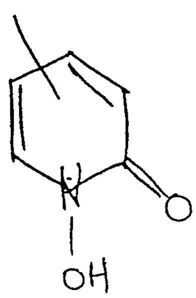

(II)

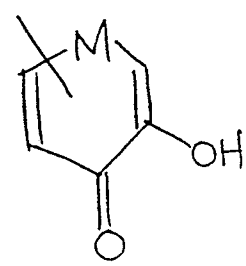

(III)

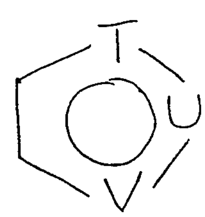

(IV)

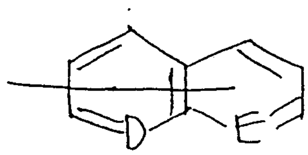

(V)

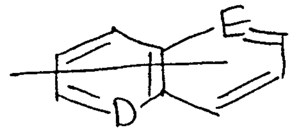

(VI)

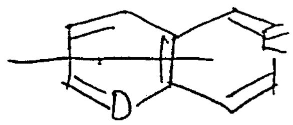

(VII)

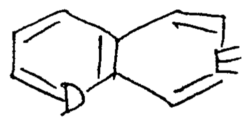

(VIII)

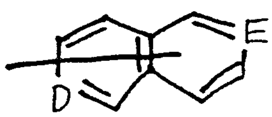

(IX)

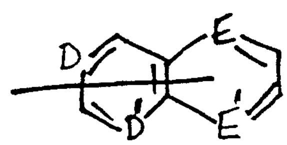

(X)

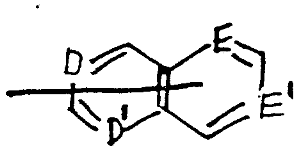

(XI)

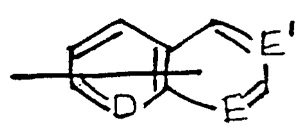

(XII)

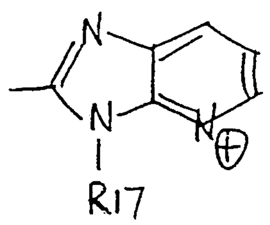

(XIII)

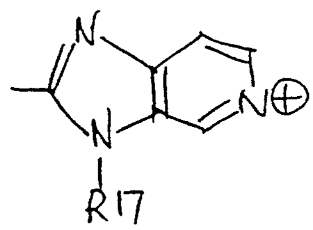

(XIV)

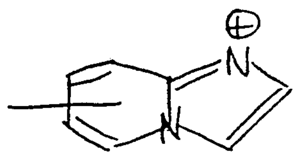

(XV)

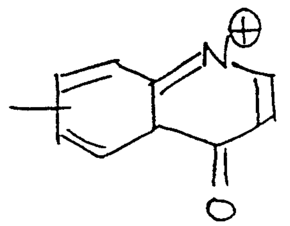

(XVI)

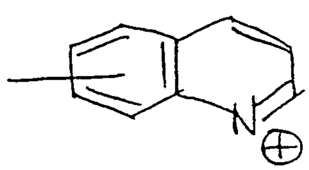

(XVII)

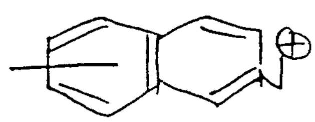

(XVIII)

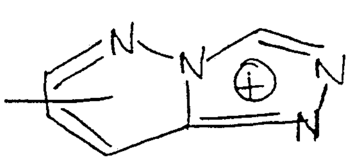

(XIX)

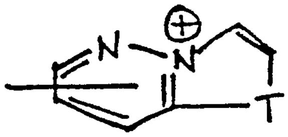

(XX)

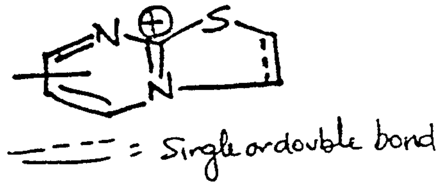


(XXI)

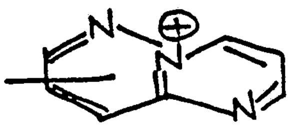

(XXII)

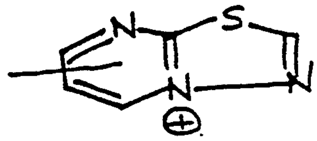

(XXIII)

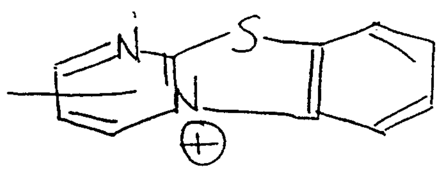

(XXIV)

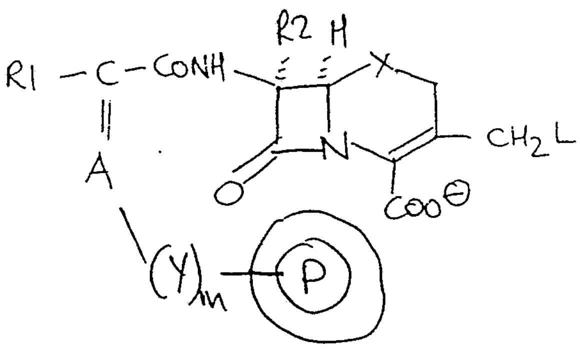

(XXV)

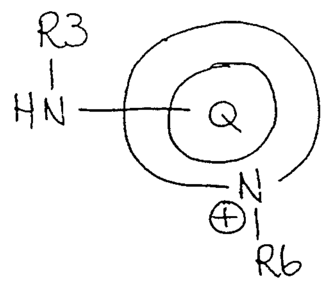

(XXVI)

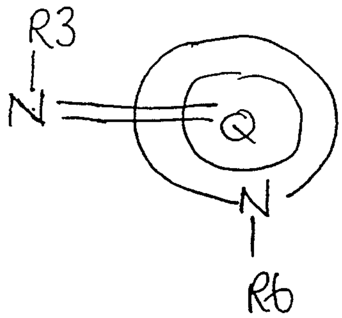

(XXVII)

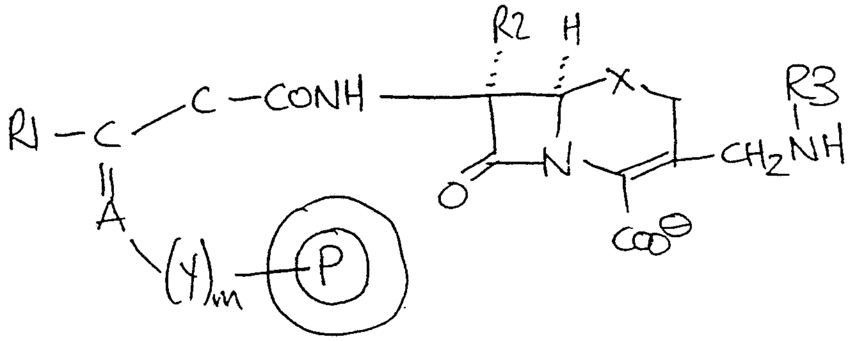

(XXVIII)

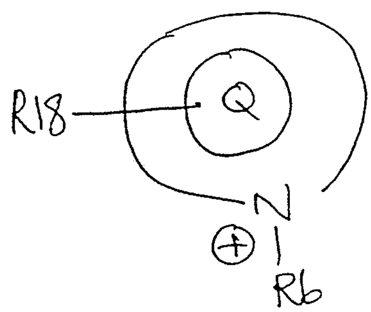

(XXIX)

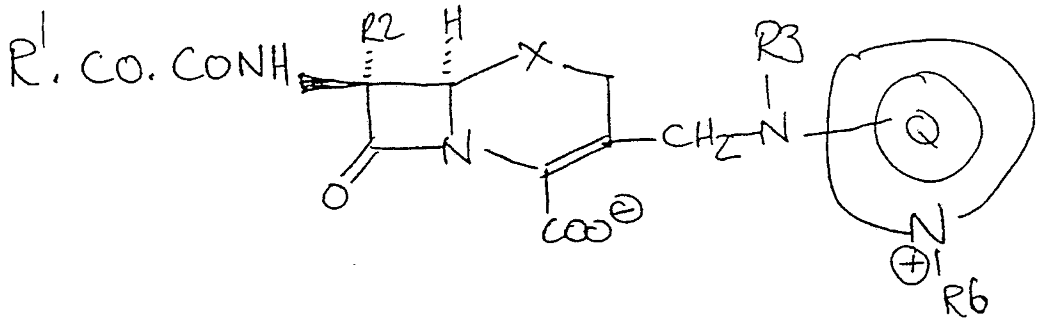

(XXXIV)

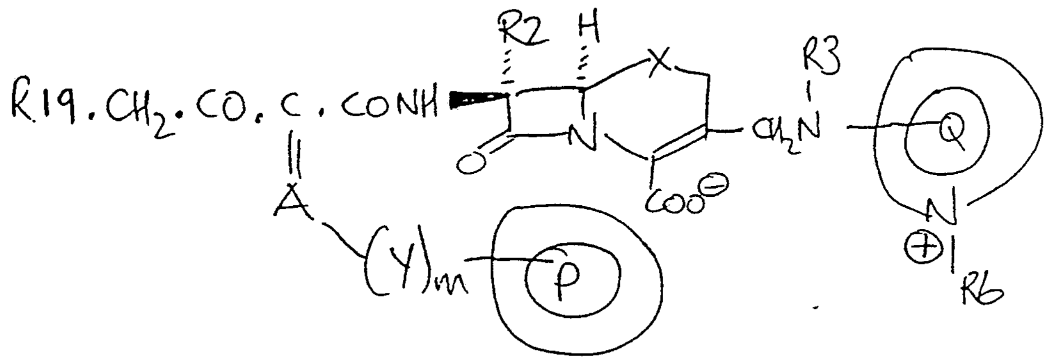

(XXXV)

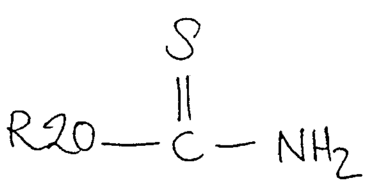

(XXXVI)

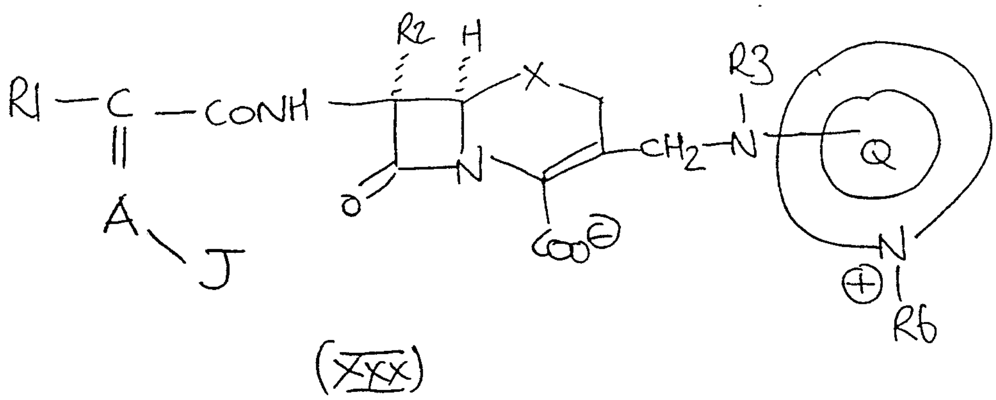

(XXX)

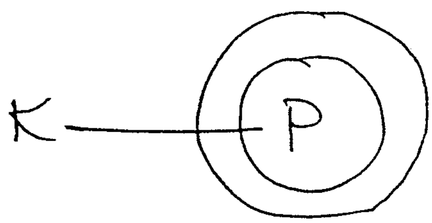

(XXXI)

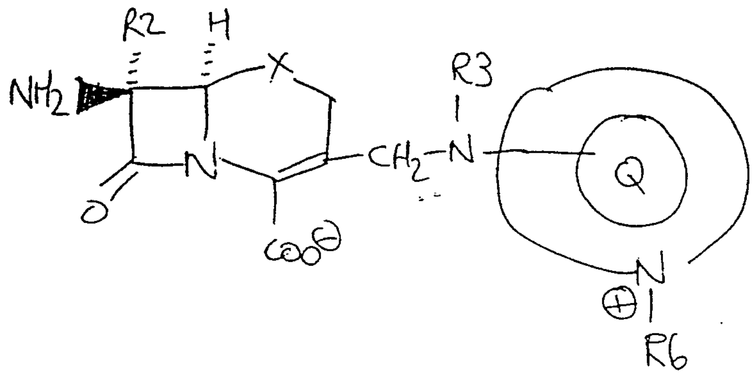

(XXXII)

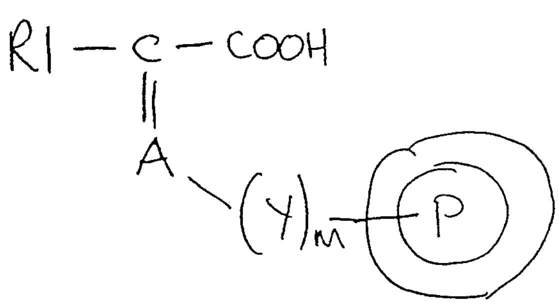

(XXXIII)